# EUROPEAN PATENT SPECIFICATION

(11) **EP 3 896 151 B1**
(45) Date of publication and mention of the grant of the patent: **11.11.2026**
(21) Application number: 18942982.2
(22) Date of filing: 12.12.2018
(51) Int. Cl.: C12N 1/205, C12N 1/20, A61K 35/747, A61P 29/00, C12R 1/25

(54) **USE OF LACTOBACILLUS PLANTARUM TWK10 IN PREPARATION OF COMPOSITION FOR RESISTANCE TO POST-EXERCISE INFLAMMATION OR FOR REDUCING BODY FAT**
VERWENDUNG VON LACTOBACILLUS PLANTARUM TWK10 BEI DER HERSTELLUNG EINER ZUSAMMENSETZUNG FÜR RESISTENZ GEGEN ENTZÜNDUNG NACH DEM TRAINING ODER ZUR VERMINDERUNG VON KÖRPERFETT
UTILISATION DE LACTOBACILLUS PLANTARUM TWK10 DANS LA PRÉPARATION D'UNE COMPOSITION POUR UNE RÉSISTANCE À UNE INFLAMMATION POST-EXERCICE OU POUR RÉDUIRE LA GRAISSE CORPORELLE

(43) Date of publication of application: 20.10.2021
(73) Proprietor: Synbio Tech Inc., Kaohsiung City, Taiwan 821 (TW)
(72) Inventor: HUANG, Chi-Chang, Taoyuan City, Taiwan 333 (CN); HUANG, Wen-Ching, Chongqing S. Rd., Zhongcheng Dist. Taipei City, Taiwan 100 (CN); LIN, Jin-Sheng, Kaohsiung City, Taiwan 821 (CN); LEE, Mon-Chien, Taoyuan City, Taiwan 333 (CN); NG, Ker-Sin, Kaohsiung City, Taiwan 821 (CN)
(74) Representative: Cabinet Chaillot
(86) International application number: PCT/CN2018/120722
(87) International publication number: WO 2020/118576

(56) References cited:
- CN-A- 101 325 960
- CN-A- 103 655 637
- CN-A- 104 894 002
- CN-A- 108 624 523
- US-A1- 2018 055 893
- US-A1- 2018 264 054
- WEN-CHING HUANG: "Effect of Lactobacillus Plantarum TWK10 on Improving Endurance Performance in Humans", CHINESE JOURNAL OF PHYSIOLOGY, vol. 61, no. 3, 30 June 2018 (2018-06-30), CN, pages 163 - 170, XP055770695, ISSN: 0304-4920, DOI: 10.4077/CJP.2018.BAH587
- YI-MING CHEN ET AL: "Lactobacillus plantarum TWK10 Supplementation Improves Exercise Performance and Increases Muscle Mass in Mice", NUTRIENTS, vol. 8, no. 4, 7 April 2016 (2016-04-07), pages 205, XP055767589, DOI: 10.3390/nu8040205
- NISHIMURA MIE ET AL: "Effects of yogurt containing Lactobacillus plantarum HOKKAIDO on immune function and stress markers", JOURNAL OF TRADITIONAL AND COMPLEMENTARY MEDICINE, vol. 6, no. 3, 1 July 2016 (2016-07-01), INDIA, pages 275 - 280, XP055921638, ISSN: 2225-4110, DOI: 10.1016/j.jtcme.2015.07.003
- JUNG SAEM ET AL: "Supplementation with two probiotic strains, Lactobacillus curvatus HY7601 and Lactobacillus plantarum KY1032, reduced body adiposity and Lp-PLA2 activity in overweight subjects", JOURNAL OF FUNCTIONAL FOODS, vol. 19, 1 December 2015 (2015-12-01), NL, pages 744 - 752, XP055922382, ISSN: 1756-4646, DOI: 10.1016/j.jff.2015.10.006

## Description

### FIELD OF THE INVENTION

The present invention belongs to the application field of probiotics, and more particularly relates to *Lactobacillus plantarum* that can be used for treating chronic inflammation and obesity.

### DESCRIPTION OF THE PRIOR ART

Probiotics are very popular in the market of healthy foods, because the source of probiotics is natural and harmless to the human body, the threshold for taking them is low, and they provide various regulating effects on human body's health. Among them, *Lactobacillus plantarum* is a common probiotic, a Gram-positive bacterium, which can be found in fermented foods or animal saliva. It has the largest genome in *Lactobacilli* and has many variations, so it often demonstrates different characteristics and effects among the same type of strain due to the extent of variation and the condition of adaptation.

Previous reports have indicated that *Lactobacillus plantarum* has a variety of medical uses, including protection of respiratory tract against viral infections, increasing the sensitivity of cancer cells to chemotherapeutic drugs, as well as an additive for various foods, for example, for preventing the growth of toxic fungi in corns.

Inflammation refers to the defensive-based response of live tissues with vascular system to inflammatory factors and local damage, which can be divided into acute inflammation and chronic inflammation. Acute inflammation is the initial response of organisms to harmful stimuli. More plasma and leukocytes (especially granulocytes) move from the blood to the damaged tissues for a series of chain reactions involving local vascular system, immune system and individual cell in the damaged tissues. Chronic inflammation leads to changes in cell types in the inflamed area, and causes synchronous tissue destruction and repair. In addition, human adipocyte has the ability to secrete inflammatory hormones, so excessively high body adipocyte is also a factor that causes chronic inflammation of the body. In some special cases, inflammation may even cause allergies in the human body's immune system, which in turn attacks its own tissues and cells, such as rheumatoid arthritis, lupus erythematosus and other immune system allergies. Long-term chronic inflammation can cause a series of diseases and make the organs and tissues gradually lose function, not only cause fatigue, aging, hormonal imbalance easily, but also lead to a variety of serious diseases, including hepatitis, pneumonia, nephritis, autoimmune diseases, arthritis, diabetes, Alzheimer's disease, cardiovascular disease, and even cancers.

Injury to muscle fibers or connective tissues from exercise can cause muscle inflammation, accompanied by ache, pain, various discomforts, and chronic inflammation caused by long-term accumulation, which not only discourage people from exercising and reduce quality of life, it also causes the human body to be prone to further sports injuries and posture changes. Especially for athletes, how to effectively reduce inflammatory conditions after exercise is a key factor to improve the efficiency of practice and competition and reduce sports injuries.

Although there are a lot of health foods on the market in Taiwan, only five have been certified by Taiwan Ministry of Health and Welfare to have anti-inflammatory effects (Table 1), with the same purpose of adjusting allergic physique and antianaphylaxis. However, the mechanism of allergies is induced by allergens, resulting in immunoglobulin to over-stimulate the sensitization of mast cells; unlike acute or chronic inflammation after exercise, which is mainly caused by injury on the tendon or ligament. At present, the application of probiotics to improve inflammation after exercise remains to be studied and researched.

**Table 1 - List of anti-inflammatory health foods approved by Taiwan Food and Drug Administration of the Ministry of Health and Welfare.**

| **License Number** | **Product Name** | **Health Caring Effects** | **Applicant** |
|---|---|---|---|
| Health Food License No. A00116 by Taiwan Department of Health | Stolle Essential (Powder Food) | Auxiliary adjustment of allergic physique, immunomodulatory function | LanFar International Co., Ltd. |
| Health Food License No. A00128 by Taiwan Department of Health | Yakult 300LIGHT Live Bacterial Fermented Milk | Auxiliary adjustment of allergic physique, immunomodulatory | Yakult Co., Ltd. |
| | | function, improvement of gastrointestinal function | |
| Health Food License No. A00218 by Taiwan Department of Health | Baiyimin Probiotic Capsule | Auxiliary adjustment of allergic physique | Syngen Biotech Co., Ltd. |
| Health Food License No. A00322 by Taiwan Department of Health | L-137tment oLactobacilli Capsule | Auxiliary adjustment of allergic physique, immunomodulatory function | Chambio Co., Ltd. |
| Health Food License No. A00329 by Taiwan Department of Health | Roymin Capsule | Auxiliary adjustment of allergic physique | Standard Chem & Pharm Co., Ltd. |

Previously, related literatures disclose that *Lactobacillus plantarum* can be used to improve anti-inflammatory related diseases; however, there is no related publication of probiotics that can improve inflammation after exercise.

There is only literature disclosing that Lactobacillus plantarum strain TWK10 increases muscle mass and endurance and thus can be used to improve exercise performance (US 2018/0055893).

Therefore, how to provide a new composition for use in treating chronic inflammation and obesity is the problem to be solved in the present invention.

### SUMMARY OF THE INVENTION

The present invention provides a composition comprising *Lactobacillus plantarum* TWK10 for use in treating chronic inflammation and obesity, in an individual, wherein the *Lactobacillus plantarum* TWK10 is deposited in China General Microbiological Culture Collection Center (CGMCC) with an accession number CGMCC 13008; wherein the *Lactobacillus plantarum* TWK10 is inactivated bacteria, wherein a dose is greater than or equal to 3x10¹¹ CFU (colony forming unit)/ day; wherein the composition can reduce neutrophil to lymphocyte ratio (NLR) and platelet to lymphocyte ratio (PLR). The *Lactobacillus plantarum* TWK10 is deposited in Taiwan Food Industry Research and Development Institute (FIRDI) with the accession number BCRC910734, and is also deposited in China General Microbiological Culture Collection Center (CGMCC) with the accession number CGMCC 13008.

In order to achieve the aforementioned objective of the present invention, treating chronic inflammation and obesity by the Lactobacillus plantarum TWK10 is achieved by reducing a neutrophil to lymphocyte ratio (NLR) and a platelet to lymphocyte ratio (PLR).

In order to achieve the aforementioned objective of the present invention, the *Lactobacillus plantarum* TWK10 is a live or an inactivated bacterium, and the inactivated bacterium is inactivated by heat or subjected to other physical or chemical treatments. When the *Lactobacillus plantarum* TWK10 is a live bacterium, a dose is greater than or equal to 3x10¹⁰ CFU ( colony forming unit)/ day; wherein a dose greater than 1x10¹¹ CFU (colony forming unit) per day has better effects than 3x10¹⁰ CFU on improving inflammation after exercise.

The present invention provides a composition comprising *Lactobacillus plantarum* TWK10 for use in treating chronic inflammation and obesity, wherein a therapeutically effective amount of a probiotic composition including Lactobacillus plantarum TWK10 with the accession number CGMCC 13008 is administered.

The present disclosure further provides a composition for improving diseases caused by excessively high neutrophil to lymphocyte ratio (NLR) and platelet to lymphocyte ratio (PLR) by administering an effective amount of said composition, and the probiotic composition comprises the *Lactobacillus plantarum* TWK10 with the accession number CGMCC 13008.

The present invention provides a composition for use in treating obesity by reducing body fat in an individual, comprising administering a therapeutically effective amount of a probiotic composition including *Lactobacillus plantarum* TWK10 with the accession number CGMCC 13008.

In order to achieve the aforementioned objective, the reducing body fat is reducing whole body fat.

In order to achieve the aforementioned objective, the therapeutically effective amount comprises an administrated dosage of the *Lactobacillus plantarum* TWK10 being greater than or equal to 3x10¹⁰ CFU (colony forming unit) per day.

The present invention provides a composition for use in treating obesity , comprising administering a therapeutically effective amount of a probiotic composition including *Lactobacillus plantarum* TWK10 with the deposition number CGMCC13008.

The composition of the present invention can be used for alleviating a disease caused by excessively high body fat in an individual, comprising administering a therapeutically effective amount of a probiotic composition including *Lactobacillus plantarum* TWK10 with the deposition number CGMCC13008.

The aforementioned probiotic composition can comprise pharmaceutically acceptable carrier, excipients and diluent, and a dosage form of the composition is selected from the group consisting of solution, suspension, emulsion, powder, tablet, pill, syrup, lozenge, troche, chewing gum, jatex and capsule.

The aforementioned probiotic composition can be further made into fluid milk products, concentrated milk, yogurt, sour milk, frozen yogurt, Lactobacillus-fermented beverages, milk powder, ice cream, cream cheeses, hard cheeses, soy milk, fermented soy milk, vegetable-fruit juices, fruit juices, sports drinks, confectionery, jellies, candies, infant foods, health foods, animal feeds, Chinese medicinal herbs compositions and dietary supplements.

The aforementioned probiotic composition comprises one or any combination of the following probiotic groups: *Lactobacillus species, Lactococcus species, Pediococcus species, Leuconostoc species, Enterococcus species, Streptococcus species, Bifidobacterium species,* and *Saccharomycete species.*

An embodiment of the present invention demonstrates that the live and high-dose group (3x10¹¹ CFU/ day) and the heat-killed group of TWK10 can reduce the inflammatory index values (NLR and PLR) and help to treat chronic inflammation, and is available to be applied to diseases or symptoms caused by excessively high NLR and PLR..

Another embodiment of the present invention demonstrates that both the *Lactobacillus plantarum* TWK10 viable bacterium and the heat-killed bacteria group can reduce the body fat; moreover, it is a dose-dependent effect. *Lactobacillus plantarum* TWK10 can be used to reduce body fat, control obesity and overweight, or improve the diseases and symptoms caused by excessively high body fat, and has the potential to improve chronic inflammation.

The advantages of the new method of the probiotics provided in the present invention are as follows:
1) The objects are concise and novel
   For the first time, TWK10 is provided for improving the symptoms of inflammation after exercise, which is different from other probiotics that inhibit diseases caused by inflammation, and providing evidence of rigorous human clinical data. In addition, TWK10 also provides the effect of reducing body fat at the same time, combining anti-inflammatory and reducing body fat after exercise, especially for athletes, it can help enhance physical functions in all aspects.
2) Wide range of applications
   Because inflammation, or high body fat is the cause or evaluation factor of common diseases or symptoms, the composition in the present invention will help to improve the diseases and symptoms caused by chronic inflammation or obesity.
3) Synergy effect
   TWK10 can lower body fat, and because fat releases inflammatory hormones, long-term use TWK10 can achieve the synergistic effect of improving chronic inflammation under the original effect of improving inflammation after exercise.

### BRIEF DESCRIPTION OF THE DRAWINGS

FIG. 1 shows the change in neutrophil to lymphocyte ratio (NLR) after exercise and administration of a probiotic composition containing *Lactobacillus plantarum* TWK10;
FIG. 2 shows the change in platelet to lymphocyte ratio (PLR) ratio after exercise and administration of the probiotic composition containing *Lactobacillus plantarum* TWK10.
FIG. 3 TWK10 can lower body fat, and because fat releases inflammatory hormones, long-term use TWK10 can achieve the synergistic effect of improving chronic inflammation under the original effect of improving inflammation after exercise.

### DETAILED DESCRIPTION OF THE INVENTION

The present invention will be further described in the following embodiments. However, the embodiments are merely representative and illustrative of the meaning and spirit of the present invention, and thus the embodiments are for exemplified instead of limitative purpose, and should not be construed as the limitations of implementing the present invention or being limited to the described embodiments or manners.

All technical and scientific terms described in the specification and claims are defined according to the following description unless otherwise defined. Among them, the singular terms "a", "an", "one", "the" can refer to more than one subject unless otherwise indicated; unless otherwise indicated, ordinary techniques employed in the implementation of the present invention can include clinical sample collection and analysis, high-efficient liquid-phase chromatography, protein chemistry, biochemistry, recombinant DNA technology, pharmacological technology; in addition, the terms "contain", "include" and "comprise" are open conjunctions; unless otherwise indicated, the materials used in the present invention are commercially available and can be easily acquired.

The term "probiotics" refers to microorganisms that provide physiological health benefits to a user when properly administered. The microorganisms are non-toxic and have intestinal adhesion, and gain effects; the term "colony forming unit (CFU)" refers to a term of total number of colonies of microorganisms such as bacteria, mold, yeast, etc. per unit volume; the term "composition" refers to a product which is derived from mixing or combining more than one active ingredient; "carrier", "excipients" refer to a compound or medicament that is non-toxic, and has the function of assisting cells or tissues to absorb drugs, and can be aromatics, buffers, binders, colorants, disintegrants, thinners, emulsifiers, extenders, flavor-improving agents, gellants, glidants, preservatives, skin-penetration enhancers, solubilizers, stabilizers, suspending agents, sweeteners, tonicity agents, viscosity-increasing agents, or any combination of the above; the term "diluent" refers to a solvent used for diluting prior to administration of the composition, and is used for stabilizing the compound. Buffer solution in the present invention is used to dissolve salt and regulate pH value, including but not limited to phosphate buffered saline (PBS); the term "effective amount" refers to a sufficient amount of a compound or drug to reduce one or more disease symptoms or physiological conditions of a patient after taking it. For example, an "effective amount" of a treatment includes a dose of a compound provided in the present invention that clinically reduces a particular physiological indicator significantly; the term "maximal oxygen uptake (abbreviated as VO₂max)" refers to a highest value of oxygen that can be consumed or utilized by tissue cells when a person is engaged in the most intense exercise above sea level; the term "endurance exercise ability test with fixed intensity and time" refers to exercise testing on a treadmill or bicycle with an intensity of 60% VO₂max or more for at least 30 minutes.

The following embodiments are merely illustrative of the probiotic composition used in the present invention, wherein the probiotics can be live or inactivated, and the inactivated bacteria are inactivated by heat or subjected to other physical or chemical treatments.

The *Lactobacillus plantarum* strain TWK10 (hereinafter referred to as TWK10) used in the present invention was obtained by separating from fermented cabbages in Taiwan by Dr. Tsung-Yu Tsai, and is deposited in China General Microbiological Culture Collection Center (CGMCC) with the accession number CGMCC 13008, and the juridical association of Taiwan Food Industry Research and Development Institute (FIRDI) with the accession number BCRC910734.

### Embodiment 1: Anti-inflammatory effects of Lactobacillus plantarum TWK10

### a) Experimental design and process

The experiment employs a double-blind cross-over trial. The subjects were between 20-40 years old. After a two-week wash period, they were administrated by the probiotics for six weeks. The subjects were divided into 5 groups, 16 people in each group, according to the result of a maximum oxygen uptake (VO₂max, mL/kg/min), and an endurance exercise test with fixed intensity at the beginning and end of the administration is executed. Furthermore, a blood test to determine changes of physiological factors was also performed. The probiotic composition of each group was taken three times a day, one capsule at a time, and a dose of administration and strain of each group are listed as follows:
(1) Placebo group: TWK10-free placebo capsules (containing maltodextrin, lactose, microcrystalline α-cellulose), 3 capsules a day, take one capsule after each of three meals with warm water.
(2) Low-dose group: recommended 1 time dose of TWK10 live bacteria group (TWK10-L): One TWK10 capsule containing 1x10¹⁰ CFU, 3 capsules per day, 3x10¹⁰ CFU/ day, take one capsule after each of three meals with warm water.
(3) Middle-dose group: recommended 3 times dose of TWK10 live bacteria group (TWK10-M): One TWK10 capsule containing 3x10¹⁰ CFU, 3 capsules per day, 9x10¹⁰ CFU/ day, take one capsule after each of three meals with warm water.
(4) High-dose group: recommended 10 times dose of TWK10 live bacteria group (TWK10-H): One TWK10 capsule containing 1x10¹¹ CFU, 3 capsules per day, 3x10¹¹ CFU/ day, take one capsule after each of three meals with warm water.
(5) TWK10 heat-killed bacteria group (TWK10-HK): One TWK10 heat-killed bacteria capsule containing 1x10¹¹ CFU, 3 capsules per day, 3x10¹¹ CFU/ day, take one capsule after each of three meals with warm water.

### b) Results

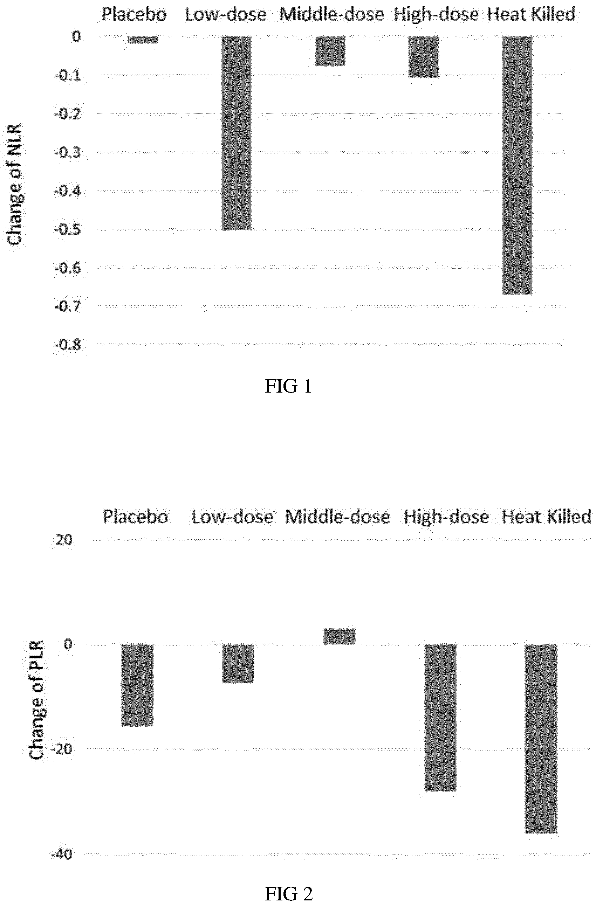

After analysis of six weeks that the subjects had performed the endurance exercise test with fixed intensity and time (120 minutes), the change was represented in neutrophil to lymphocyte ratio (NLR) and platelet to lymphocyte ratio (PLR) in the respective groups, both of which representing the state of the lymphatic system in the body and being indicators for measuring inflammation in the body. A decrease in a ratio indicates an improvement in inflammation, and there are even reports indicating that NLR or PLR can be used as an observation factor for cancer metastasis or other diseases. As shown in the results in FIG. 1 and FIG. 2, the vertical axis indicates the amount of the change in NLR or PLR relative to the start of the test.

The results show that High-dose, Medium-dose, Low-dose groups and TWK10 heat-killed bacteria group can reduce the NLR. Among the change of NLR, the low-dose and heat-killed bacteria groups decreased by about 30-40 times compared with the control group. As for PLR, the High-dose and TWK10 heat-killed bacteria groups decreased by 1.2-1.8 times compared with the control group. Overall, taking TWK10 dead bacteria can improve inflammation after exercise, and daily dose of TWK10 live bacteria of more than 3x10¹⁰ CFU also has effects of improving inflammation after exercise, if taken higher dose per day, which is more than 9x10¹⁰ CFU live bacteria, for example, 1x10¹¹ or 3x10¹¹ CFU, better effects can be achieved.

### Embodiment 2: Effects of the Lactobacillus plantarum TWK10 on reducing body fat

Because fat cells secrete inflammatory hormones and cause systemic inflammation, high body fat is also closely related to chronic inflammation. Continued, we will explore whether TWK10 can reduce body fat in addition to improving the symptoms of inflammation after exercise.

### a) Experimental design and process are same as embodiment 1.

The experiment employs a double-blind cross-over trial. The subjects were between 20-40 years old. After two weeks of washout period and one week rest, each subject was given an administration of the probiotics for six weeks. The subjects performing an endurance exercise test with fixed intensity at the beginning and end of the administration were divided into 5 groups according to a maximum oxygen uptake (VO₂ₘₐₓ, mL/kg/min), 16 people in each group, and changes in the whole body fat were measured; the probiotic composition of each group was taken three times a day, one capsule at a time, and a dose of the administration and strain of each group are same as embodiment 1.

### b) Results

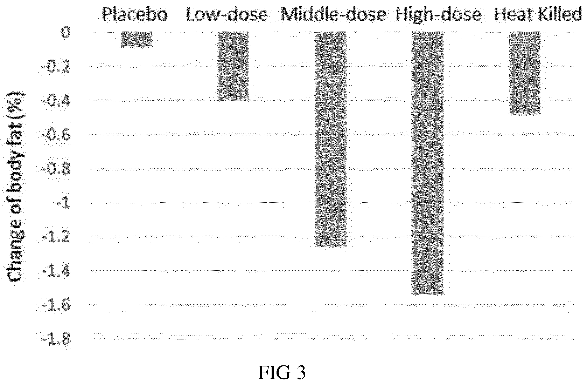

After taking the probiotic composition for six weeks, changes in body fat were analyzed after each of the testes had performed the endurance exercise test with fixed intensity and time. The results are shown in figure 3; the vertical axis indicates changes of body fat percentages measured at the end of the administration relative to the start of the administration.

The results show that both the live bacteria and heat-killed bacteria groups can reduce body fat, among them, the low-dose and heat-killed bacteria groups decreased by about 0.5% compared with the control group. As the dose increases, the body fat declines more significantly in the live bacteria group, and the high-dose group can make body fat decreased by 1.5%, indicating that TWK10 has the effect of reducing body fat, whether it is in the form of live or non-activated bacteria.

In summary, TWK10 has the effects of reducing inflammation after exercise and reducing body fat. In addition, since TWK10 can reduce NLR or (and) PLR, NLR and PLR can also be used as indicators and causes for the diagnosis of metastasis and infections of many cancers and diseases. Since excessively high body fat is one of the causes of systemic chronic inflammation, chronic diseases, cardiovascular diseases etc., implementation of any use of the results of the present invention to improve related diseases or symptoms caused by excessively high NLR, or PLR, or excessively high body fat shall be deemed to be included in the scope of the claims of the present invention.

Due to the synergistic effect of reducing body fat and improving inflammation, long-term use of the probiotic composition of the present invention can not only treat obesity, but also simultaneously reduce systemic chronic inflammation, achieving a dual and additive body improving effect.

A dosage form of the probiotic composition used in the present invention comprises, but is not limited to, solution, emulsion, suspension, powder, tablet, pill, lozenge, troche, chewing gum, capsule, and other similar or suitable dosage forms.

The probiotic composition used in the present invention can optionally comprises other bacteria species taught in the general knowledge in the technical field of the present invention: *Lactobacillus sp., Lactococcus sp., Pediococcus sp., Leuconostoc sp., Enterococcus sp., Streptococcus sp., Bifidobacterium sp.,* yeasts (*Saccharomyces sp*.).

The probiotic composition used in the present invention can be a food composition, and further made into capsule, tablet, beverages, powder or dairy products; the aforementioned composition used in the present invention can further be added with an edible material to prepare as a food product or health caring product, wherein the edible material comprises, but is not limited to: water, fluid milk products, milk, concentrated milk, fermented milk (such as yogurt, sour milk, frozen yogurt, lactic acid bacteria-fermented beverages), milk powder, ice cream, cream cheeses, dry cheeses, soybean milk, fermented soybean milk, vegetable-fruit juices, fruit juices, sports drinks, confectionery, jellies, candies, infant formulas, health foods, animal feeds, Chinese medicinal herbs, and dietary supplements.

The aforementioned composition used in the present invention can be a dietary supplement which can be administered to a user in the following manners: mixed with a suitable drinkable liquid, such as water, yoghurt, milk or fruit juices; or can be mixed with a solid or liquid food. The form of the dietary supplement can be in an administrated form of tablet, pill, capsule, lozenge, granule, powder, suspending agent, sachet, soft tablet, candy, bar, syrup and corresponding administration forms, generally in the form of dose unit and is manufactured by conventional methods for preparing dietary supplements.

In the embodiments of the present invention, an effective dose varies depending on the combination therapy. The combination therapy can further include periodic treatments that vary depending on factors such as co-administered compositions, diseases, physiological abnormalities, physiological conditions, and the like.

The embodiments described above are merely for explaining the technical ideas and characteristics of the present invention, and the purpose thereof is to enable those skilled in the art to understand the contents of the present invention and implement them accordingly.

## Claims

1. A composition comprising *Lactobacillus plantarum* TWK10 for use in treating chronic inflammation and obesity, in an individual, wherein the *Lactobacillus plantarum* TWK10 is deposited in China General Microbiological Culture Collection Center (CGMCC) with an accession number CGMCC 13008; wherein the *Lactobacillus plantarum* TWK10 is inactivated bacteria, wherein a dose is greater than or equal to 3x10¹¹ CFU (colony forming unit)/ day; wherein the composition can reduce neutrophil to lymphocyte ratio (NLR) and platelet to lymphocyte ratio (PLR).

2. The composition for use according to claim 1, wherein the *Lactobacillus plantarum* TWK10 is inactivated by heat or subjected to other physical or chemical treatments.

3. The composition for use according to claim 1, the composition comprising pharmaceutically acceptable carrier, excipients and diluent.

4. The composition for use according to claim 1, wherein a dosage form of the composition is selected from the group consisting of solution, suspension, emulsion, powder, tablet, pill, syrup, lozenge, troche, chewing gum, jatex and capsule.

5. The composition for use according to claim 1, wherein the composition is made into fluid milk products, concentrated milk, yogurt, sour milk, frozen yogurt, Lactobacillus-fermented beverages, milk powder, ice cream, cream cheeses, hard cheeses, soy milk, fermented soy milk, vegetable-fruit juices, fruit juices, sports drinks, confectionery, jellies, candies, infant foods, health foods, animal feeds, Chinese medicinal herbs compositions and dietary supplements.

6. The composition for use according to claim 1, wherein the composition comprises one or any combination of the following probiotic groups: *Lactobacillus species, Lactococcus species, Pediococcus species, Leuconostoc species, Enterococcus species, Streptococcus species, Bifidobacterium species,* and *Saccharomyces species.*

## Patentansprüche

1. Zusammensetzung, umfassend *Lactobacillus plantarum* TWK10 zur Verwendung bei der Behandlung von chronischer Entzündung und Fettleibigkeit bei einem Individuum umfasst, wobei der *Lactobacillus plantarum* TWK10 im China General Microbiological Culture Collection Center (CGMCC) unter der Zugangsnummer CGMCC 13008 deponiert ist; wobei der *Lactobacillus plantarum* TWK10 inaktivierte Bakterien sind, wobei eine Dosis größer als oder gleich wie 3×10¹¹ KBE (koloniebildende Einheiten)/Tag ist; wobei die Zusammensetzung das Neutrophilen-Lymphozyten-Verhältnis (NLR) und das Thrombozyten-Lymphozyten-Verhältnis (PLR) reduzieren kann.

2. Zusammensetzung zur Verwendung nach Anspruch 1, wobei der *Lactobacillus plantarum* TWK10 durch Hitze inaktiviert ist oder anderen physikalischen oder chemischen Behandlungen unterzogen wird.

3. Zusammensetzung zur Verwendung nach Anspruch 1, wobei die Zusammensetzung einen pharmazeutisch annehmbaren Träger, Hilfsstoffe und ein Verdünnungsmittel umfasst.

4. Zusammensetzung zur Verwendung nach Anspruch 1, wobei eine Darreichungsform der Zusammensetzung ausgewählt ist aus der Gruppe, bestehend aus Lösung, Suspension, Emulsion, Pulver, Tablette, Pille, Sirup, Lutschtablette, Pastille, Kaugummi, Jatex und Kapsel.

5. Zusammensetzung zur Verwendung nach Anspruch 1, wobei die Zusammensetzung zu flüssigen Milchprodukten, Kondensmilch, Joghurt, Sauermilch, gefrorenem Joghurt, mit Lactobacillus fermentierten Getränken, Milchpulver, Speiseeis, Frischkäse, Hartkäse, Sojamilch, fermentierter Sojamilch, Gemüse-Fruchtsäften, Fruchtsäften, Sportgetränken, Süßwaren, Gelees, Bonbons, Säuglingsnahrung, Gesundheitsnahrung, Futtermitteln, chinesischen Kräuterzusammensetzungen und Nahrungsergänzungsmitteln verarbeitet wird.

6. Zusammensetzung zur Verwendung nach Anspruch 1, wobei die Zusammensetzung eine oder eine beliebige Kombination der folgenden probiotischen Gruppen umfasst: *Lactobacillus-Spezies, Lactococcus-Spezies, Pediococcus-Spezies, Leuconostoc-Spezies, Enterococcus-Spezies, Streptococcus-Spezies, Bifidobacterium-Spezies* und *Saccharomyces-Spezies*.

## Revendications

1. Composition comprenant *Lactobacillus plantarum* TWK10 pour une utilisation dans le traitement d'une inflammation chronique et de l'obésité, chez un individu, dans laquelle le *Lactobacillus plantarum* TWK10 est déposé au Centre Général de Collection de Cultures Microbiologiques de Chine (CGMCC) sous le numéro d'enregistrement CGMCC 13008 ; dans laquelle le *Lactobacillus plantarum* TWK10 consiste en bactéries inactivées, une dose étant supérieure ou égale à 3×10¹¹ CFU (unité de formation de colonies) par jour ; dans laquelle la composition peut réduire le rapport neutrophiles/lymphocytes (NLR) et le rapport plaquettes/lymphocytes (PLR).

2. Composition pour l'utilisation selon la revendication 1, dans laquelle le *Lactobacillus plantarum* TWK10 est inactivé par la chaleur ou soumis à d'autres traitements physiques ou chimiques.

3. Composition pour l'utilisation selon la revendication 1, la composition comprenant un support, des excipients et un diluant pharmaceutiquement acceptables.

4. Composition pour l'utilisation selon la revendication 1, dans laquelle une forme posologique de la composition est choisie dans le groupe consistant en solution, suspension, émulsion, poudre, comprimés, pilules, sirop, pastilles, tablettes, gomme à mâcher, jatex et capsules.

5. Composition pour l'utilisation selon la revendication 1, dans laquelle la composition est transformée en produits laitiers fluides, lait concentré, yaourt, lait caillé, yaourt glacé, boissons fermentées au Lactobacillus, lait en poudre, crème glacée, fromages à la crème, fromages à pâte dure, lait de soja, lait de soja fermenté, jus de fruits et légumes, jus de fruits, boissons pour sportifs, confiseries, gelées, bonbons, aliments pour nourrissons, aliments diététiques, aliments pour animaux, compositions à base de plantes médicinales chinoises et compléments alimentaires.

6. Composition pour l'utilisation selon la revendication 1, dans laquelle la composition comprend un ou toute combinaison des groupes probiotiques suivants : espèces de *Lactobacillus*, espèces de *Lactococcus*, espèces de *Pediococcus*, espèces de *Leuconostoc*, espèces d'*Enterococcus*, espèces de *Streptococcus*, espèces de *Bifidobacterium* et espèces de *Saccharomyces*.
